# NEUE EUROPÄISCHE PATENTSCHRIFT

(11) **EP 1 501 453 B2**
(45) Veröffentlichungstag und Bekanntmachung des Hinweises auf die Entscheidung über den Einspruch: **30.06.2010**
(45) Hinweis auf die Patenterteilung: 07.12.2005
(21) Anmeldenummer: 04732110.4
(22) Anmeldetag: 11.05.2004
(51) Int. Cl.: A61F 2/30, A61F 2/46, A61F 2/44

(54) **HÖHENVERSTELLBARES IMPLANTAT ZUM EINSETZEN ZWISCHEN WIRBELKÖRPERN UND HANDHABUNGSWERKZEUG**
HEIGHT-ADJUSTABLE IMPLANT TO BE INSERTED BETWEEN VERTEBRAL BODIES AND CORRESPONDING HANDLING TOOL
IMPLANT REGLABLE EN HAUTEUR DESTINE A ETRE INSERE ENTRE DES VERTEBRES ET OUTIL DE MANIPULATION

(30) Priorität: 14.05.2003 DE 10321534
(43) Veröffentlichungstag der Anmeldung: 02.02.2005
(73) Patentinhaber: Kraus, Kilian, 97440 Werneck (DE)
(72) Erfinder: KRAUS, Kilian, 97440 Werneck (DE); SAAL, Norbert, 97711 Thundorf (DE)
(74) Vertreter: Grünecker, Kinkeldey, Stockmair & Schwanhäusser Anwaltssozietät
(86) Internationale Anmeldenummer: PCT/EP2004/005046
(87) Internationale Veröffentlichungsnummer: WO 2004/100837

(56) Entgegenhaltungen:
- WO-A-01/72246
- DE-A- 19 622 827
- DE-A1- 10 065 232
- DE-C- 10 127 924
- JP-A- 2 261 446
- US-A- 5 665 122
- US-A1- 2003 045 877
- US-A1-2002/0 082 695
- US-D- 4 501 22S

## Beschreibung

Die Erfindung betrifft ein.höhenverstellbares Implantat zum Einsetzen zwischen Wirbelkörpern.

Ein beispielsweise aus DE 196 22 827 A1 bekanntes Implantat weist ein erstes und ein zweites Hülsenteil auf, wobei das zweite Hülsenteil ein Außengewinde trägt, das mit einem Längsabschnitt im ersten Hülsenteil drehfixiert und axial beweglich einliegt und an seinem aus dem ersten Hülsenteil heraus ragenden Längsabschnitt von einer in das Außengewinde eingreifenden Mutter umfasst ist. Durch Drehen der Mutter wird das zweite Hülsenteil aus dem ersten Hülsenteil heraus bewegt. Zur Betätigung der Mutter wird ein stabförmiges Handhabungswerkzeug mit seinem Freiende in eine Ausnehmung am Außenumfang der Mutter eingesteckt. Durch eine Schwenkbewegung des Werkzeugs in einer quer zur Längsachse des Implantats verlaufenden Ebene wird die Mutter um ein dem Schwenkwinkel des Werkzeugs entsprechendes Stück weitergedreht. Danach wird das Werkzeug' aus der Mutter heraus gezogen und der geschilderte Vorgang so lange wiederholt, bis das Implantat die erforderliche Höhe aufweist. Während der Drehbetätigung der Mutter darf sich das erste Hülsenteil nicht mitdrehen, so dass dieses im Regelfall mit einem weiteren Werkzeug festgehalten werden muss. Die Höhenverstellung bei dem bekannten Implantat erfordert somit einen erhöhten Zeit- und gerätetechnischen Aufwand.' Außerdem ist eine relativ große Operationsöffnung erforderlich, um die genannten Manipulationen behinderungsfrei vornehmen zu können

Aus US 2003/045877A ist ein höhenverstellbares Implantat gemäß dem Oberbegriff des Patentanspruch 1 bekannt.

Die DE 100 65 232 A1 zeigt in den Figuren 2 - 8 ein höhenverstellbares Implantat (10) zum Einsetzen zwischen Wirbelkörpern, mit einem ersten und einem zweiten Hülsenteil (2, 3), wobei das zweite Hülsenteil (3) ein Außengewinde (6) trägt, das mit einem Längsabschnitt im ersten Hülsenteil (2) drehfixiert und axial beweglich einliegt nämlich durch das Zusammenwirken der Führungsschlitze (8) mit den Stiften (9), und an seinem aus dem ersten Hülsenteil (2) herausragenden Längsabschnitt von einer in das Außengewinde (6) eingreifenden Mutter (4), die einen Zahnring (7) trägt, umfasst ist.

Druckschrift JP 2-261446 zeigt ein höhenverstellbares Implantat zum Einsetzen zwischen Wirbelkörper mit zwei Implantatteilenn 9, 14. deren Höhenverstelloarkeit gewährleistet ist durch ein dem einen Implantatteil 14 zugeordnetes Außengewinde 10, in das ein eine Kegelradverzahnung aufweisender Längenanpassungsring 19 eingreift. Dieser mit der Mutter gleichzusetzende Längenanpassungsring 19 ist mit einem axial wirksamen Stopper (20) am ersten Hülsenteil fixiert, der die Bewegung des Längenanpassungsringes (19), also der Mutter, in die Längsrichtung beschränkt.

Davon ausgehend ist es die Aufgabe der Erfindung, ein Implantat und ein Handhabungswerkzeug vorzuschlagen, mit denen das Einsetzen in die Wirbelsäule erleichtert ist.

Diese Aufgabe wird durch den Gegenstand des Patentanspruchs 1 gelöst.

Gemäß Anspruch 1 trägt die Mutter einen Zahnkranz, ist also als Zahnrad ausgebildet. Mit Hilfe eines Werkzeugs mit einem geeigneten Gegenelement, beispielsweise einem Antriebszahnrad lässt sich die Höhe des Implantats verstellen, wobei mit dem Handhabungswerkzeug keine Schwenkbewegungen ausgeführt werden müssen. Außerdem ist es nicht erforderlich, das Werkzeug vielfach umzusetzen. Es kann vielmehr während des gesamten Vorgangs der.Höhenverstellung in ein und derselben Stellung gehalten werden, was die Operation wesentlich vereinfacht und Operationszeit spart.

Bei einer bevorzugten Ausgestaltung trägt die Mutter den Zahnring an ihrer dem ersten Hülsenteil zugewandten Unterseite. Die Betätigung der Mutter kann dadurch mit einem Antriebszahnrad erfolgen, dessen Drehachse rechtwinklig zur Drehachse der Mutter verläuft. Eine Umlenkung der Drehbewegung der Achse, wie dies bei einem Antriebszahnrad mit parallel zur Drehachse der Mutter ausgerichteter Achse der Fall wäre, ist nicht erforderlich, wodurch eine kompakte Bauweise begünstigt und der Herstellungsaufwand gesenkt wird.

Um eine Drehfixierung des ersten Hülsenteils zu gewährleisten ist an diesem eine Gewindebohrung vorhanden, an der ein die Mutter antreibendes Handhabungswerkzeug fixiert werden kann. Im Gegensatz zu herkömmlichen Implantaten ist somit nur ein einziges sowohl zum Antrieb der Mutter als auch zur Fixierung des ersten Hülsenteils dienendes Werkzeug erforderlich.

Bei dem aus DE 196 22 827 A1 bekannten Implantat stützt sich die Mutter beim Ausfahren des zweiten Hülsenteils an der ihr zugewandten Stirnseite des ersten Hülsenteils ab. Ansonsten ist die Mutter aber nicht am ersten Hülsenteil fixiert. Die Höhe des bekannten Implantats kann daher nur vergrößert, nicht aber verkleinert werden, d.h. das zweite Hülsenteil kann nicht in das erste Hülsenteil hineingefahren werden. Beim erfindungsgemäßen Implantat dagegen ist die Mutter mit einem axialwirksamen Formschluss, also in beiden Axialrichtungen am ersten Hülsenteil fixiert, wodurch sowohl eine Vergrößerung als auch eine Verkleinerung der Implantathöhe möglich ist. Letzteres kann beispielsweise erforderlich sein, wenn nach dem Einsetzen des Implantats eine zu große Höhenverstellung vorgenommen wurde.

Bei der Erfindung weisen die beiden Hülsenteile axial verlaufende, in ihren einander zugewandten Enden ausmündende Fenster auf, wobei die zwischen zwei benachbarten Fenstern angeordneten Umfangsabschnitte in den Fenstern des jeweils anderen Hülsenteils axial verschiebbar einliegen. Gegenüber einer Anordnung, bei der zwei Hülsenteile konzentrisch ineinander greifen, hat dies zunächst den Vorteil, dass weniger Material erforderlich ist und das Implantat dadurch insgesamt leichter wird. Außerdem steht ein wesentlich größerer Innenraum zur Verfügung, der mit Knochenmaterial o.dgl. aufgefüllt werden kann. Durch das kammartig ineinander greifen der beiden Hülsenteile ist schließlich noch eine gegenseitige Drehfixierung gewährleistet.

Vorzugsweise ist zur Abstützung des Implantats an einem Wirbelkörper eine radial verbreiterte Stirnplatte vorgesehen. Bei einer bevorzugten Ausgestaltung ist diese ein separates, lösbar fixiertes Teil. Es kann dann in jedem Einzelfall die geeignete Stirnplatte, beispielsweise eine mit einer schräg zur Mittellängsachse des Implantats verlaufenden Planebene, eingesetzt werden. Wird die Stirnplatte mit Hilfe einer Schnappverbindung am Hülsenteil fixiert, ist zum einen ein sicherer Halt am Hülsenteil gewährleistet und zum anderen eine leichte Austauschbarkeit. Die erfindungsgemäße in fertigungs- und montagetechnischer Hinsicht einfache Ausgestaltung sieht vor, dass nahe der Stirnseite eine Ringnut in der Innenwandung des Hülsenteils vorhanden ist, in die an der Unterseite der Stirnplatte angeformte Rastnasen eingreifen.

Ein Handhabungswerkzeug für ein Implantat der vorbeschriebenen Art weist gemäß Anspruch 10 ein mit der Mutter zusammenwirkendes Zahnrad auf. Vorzugsweise ist am Handhabungswerkzeug eine Halteeinrichtung zum Festhalten des Implantats vorhanden. Das Zahnrad ist so angeordnet, dass seine Achse beim Antrieb der quer zur Achse des Zahnrings der Mutter verläuft. Eine Umlenkung der Drehbewegung der Antriebsachse ist, wie bereits weiter oben erwähnt, nicht erforderliche. Eine besonders platzsparende und leicht zu bedienende Anordnung sieht vor, dass der Zahnring an der Stirnseite eines Rohrabschnittes angeordnet ist und dass der Rohrabschnitt von einem Stab durchsetzt ist, dessen Freiende aus dem Rohrabschnitt herausragt und in die Gewindebohrung des ersten Hülsenteils einschraubbar ist. Die koaxiale Anordnung des Rohrabschnittes und des Stabes ermöglicht ein kompaktes und leicht bedienbares Handhabungswerkzeug.

Die Erfindung wird nun anhand der beigefügten Zeichnungen näher erläutert. Es zeigen:
- Fig. 1: eine teilgeschnittene perspektivische Ansicht eines Implantats mit einem ersten Hülsenteil und einem darin axial verfahrbar einliegenden zweiten Hülsenteil, wobei sich das zweite Hülsenteil in seiner eingefahrenen Position befindet,

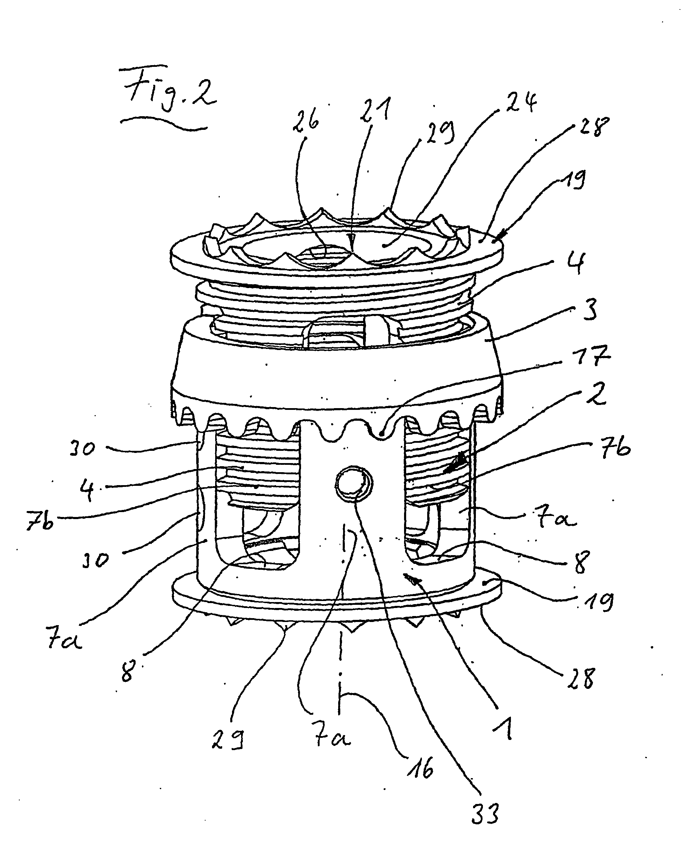

- Fig. 2: das Implantat von Fig. 1 mit teilweise ausgefahrenem zweiten Hülsenteil,

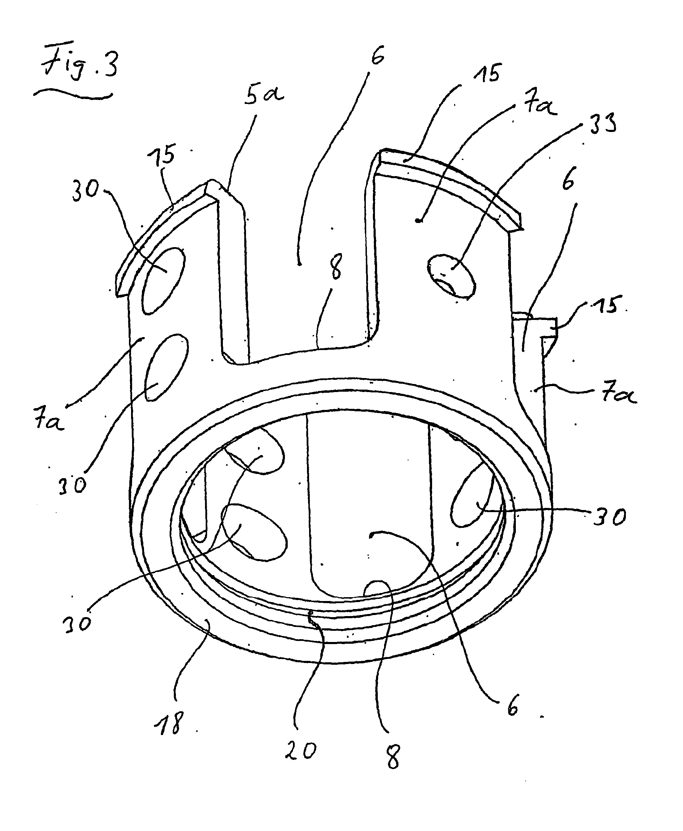

- Fig. 3: eine perspektivische Ansicht' des ersten Hülsenteils,

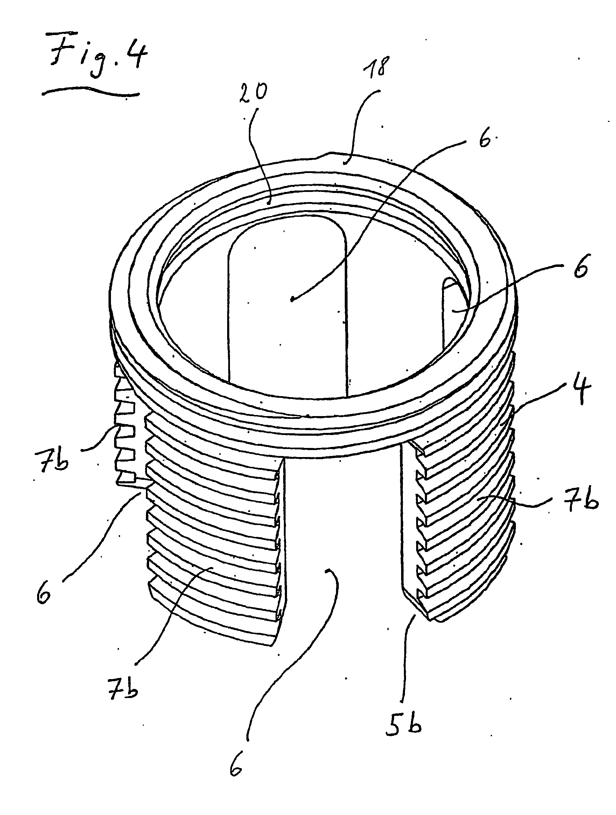

- Fig. 4: eine perspektivische Ansicht des ausfahrbaren zweiten Hülsenteils,

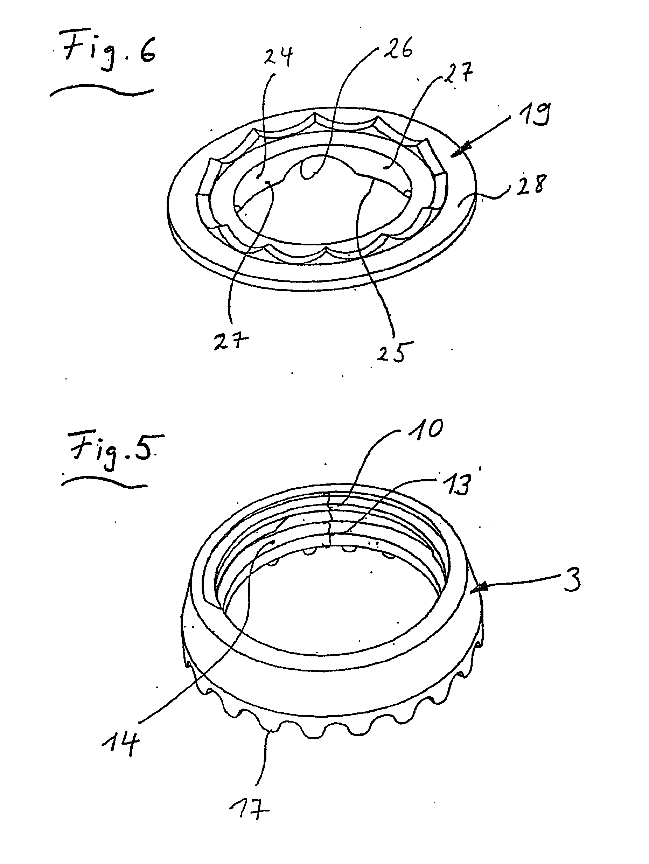

- Fig. 5: eine zum Antrieb des ersten Hülsenteils dienende Mutter,
- Fig. 6: eine an einem Hülsenteil fixierbare Stirnplatte,
- Fig. 7: ein Werkzeug zur Handhabung des Implantats.

Das in den Abbildungen gezeigte Implantat weist als Hauptbestandteile ein erstes Hülsenteil 1, ein zweites Hülsenteil 2 und eine Mutter 3 auf. Das Hülsenteil 2 ist mit einem sich im wesentlichen über seine gesamte Länge erstreckenden Außengewinde 4 versehen. Beide Hülsenteile 1,2 weisen axial verlaufende, in ihren im montierten Zustand in ihre einander zugewandten Stirnseite 5a,b ausmündende Fenster 6 auf. Die zwischen zwei benachbarten Fenstern stehen gebliebenen Umfangsabschnitte 7a,b liegen in den Fenstern 6 des jeweils anderen Hülsenteils 1,2 axial verschiebbar ein. Dabei ist das Spiel zwischen den Umfangsabschnitten 7a,b so bemessen, dass ein wackelfreier Sitz der dennoch beiden Hülsenteile ineinander und eine leichte Verschiebbarkeit gewährleistet ist. Die Hülsenteile 1,2 weisen etwa die gleiche Wandstärke und den gleichen Innendurchmesser auf, d.h. die Umfangsabschnitte 7b des zweiten Hülsenteils 2 stehen weder über den Außen- noch über den Innenumfang des Hülsenteils 1 hinaus. Die Axiallänge der Fenster 6 bzw. der Umfangsabschnitte 7a,b ist so bemessen, dass bei maximal eingefahrenem Hülsenteil 2 zwischen der Stirnseite 5a,b eines Umfangsabschnittes 7a,b und der Basis 8 eines Hülsenteils 1,2 ein Axialabstand 9 vorhanden ist.

Zur Höhenverstellung bzw. zum Ein- und Ausfahren des Hülsenteils 2 dient die Mutter 3, welche beide Hülsenteile 1,2 umfasst. Sie ist mit dein ersten Hülsenteil axial fest und drehbar verbunden. Ihre die beiden Hülsenteile 1,2 umfassende Innenseite weist einen oberen, mit einem Innengewinde 10 versehenen Längsab'schnitt und einen sich daran anschließenden gewindefreien Längsabschnitt 13 auf. In diesen ist eine Ringnut 14 eingearbeitet. Die Mutter 3 ist so am Hülsenteil 1 positioniert, dass nur der gewindefreie Längsabschnitt 13 die Umfangsabschnitte 7a des Hülsenteils 1 umfasst und das Innengewinde 10 mit dem Außengewinde 4 des Hülsenteils 2 in Eingriff steht. Nahe der Stirnseite 5a eines Umfangsabschnittes 7a ist ein kreissegmentförmiger, sich über dessen gesamte Breite erstreckender Vorsprung 15 angeformt, der in die Ringnut 14 der Mutter eingreift. Die Mutter 3 kann sich dabei zwar am Hülsenteil 1 drehen, ist aber an diesem in beiden Axialrichtungen fixiert. Die den Freienden der Umfangsabschnitte 7b zugewandte Seite der Mutter 3 trägt einen koaxial zur Mittellängsachse 16 des Implantats verlaufenden Zahnring 17. Der Zahnring 17 ist einstückig an der.Mutter 3 angeformt, beispielsweise eingefräst. Er dient dazu, die Mutter 3 mit Hilfe eines weiter unten noch näher beschriebenen Werkzeugs in Drehung zu versetzen und dadurch das Hülsenteil 2 aus dem Hülsenteil 1 heraus oder in dieses hinein zu bewegen, also die Höhe des Implantats zu verstellen.

Die den Freienden der Umfangsabschnitte 7a,b abgewandten Stirnseiten 18 tragen eine radial über den Umfang der Hülsenteile 1,2 hinaus stehende Stirnplatte 19. Die Stirnplatten 19 sind mit Hilfe einer Schnappverbindung lösbar an den Hülsenteilen 1,2 fixiert. Zu diesem Zwecke ist an der Innenseite der Hülsenteile 1,2 nähe deren Stirnseiten 18 eine Ringnut 20 vorhanden, in die an der den Stirnseiten 18a,b zugewandten Seite der Stirnplatte angeordnete Rastvorsprünge 23 eingreifen. Die Rastvorsprünge 23 sind an einer Schürze 24 angeformt, die eine zentrale Öffnung 21 in der Stirnplatte 19 umgrenzt. In den Rand 25 der Schürze 24 sind mehrere in Umfangsrichtung gleich verteilte Ausnehmungen 26 eingebracht. Die zwischen den Ausnehmungen 26 vorhandenen Umfangsabschnitte 27 tragen die Rastvorsprünge 23. Die Umfangsabschnitte 27 sind geringfügig radial nach innen elastisch auslenkbar, wodurch das Fixieren oder Entfernen der Stirnplatte 19 erleichtert ist. Auf den Außenseiten 28 der Stirnplatten 19 steht ein Zackenring 29 in Richtung der Längsachse 16 vor. Dieser dient zum Festkrallen des Implantats an einem Wirbelkörper.

In den Umfangsabschnitten 7a sind zwei Durchbrechungen 30 vorhanden. Die Durchbrechungen 30 sowie die zentrale Öffnung 21 in den Stirnplatten 19 dienen zum Einfüllen von Knochenmaterial, Knochenzement oder dgl. In einem der Umfangsabschnitte 7a ist nahe der Mutter 3 eine radial ausgerichtete Gewindebohrung 33 vorhanden. Diese dient zur Fixierung des im folgenden beschriebenen Handhabungswerkzeuges.

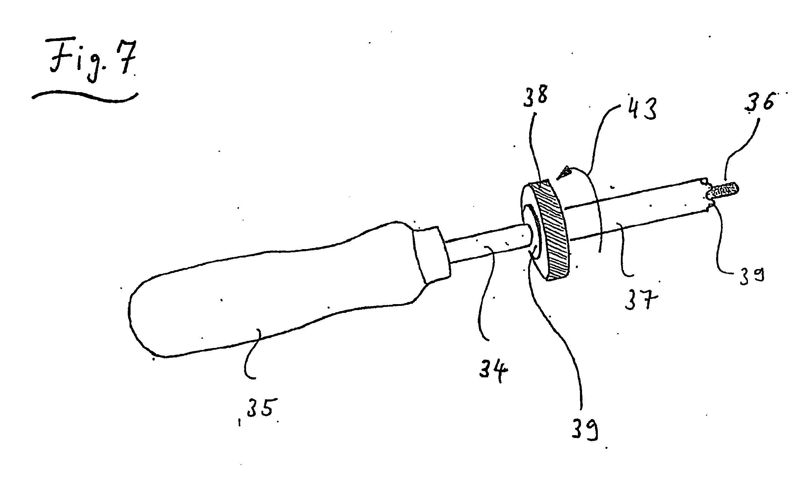

Wie aus Fig. 7 ersichtlich ist, ist dieses im wesentlichen aus einem Stab 34 gebildet, dessen eines Ende einen Griff 35 und dessen anderes Ende einen Gewindeabschnitt 36 trägt. Der sich vom Gewindeabschnitt 36 weg erstreckende Bereich des Stabes 34 ist von einer Drehhülse 37 koaxial umfasst. Das dem Griff 35 zugewandte Ende der Drehhülse 37 trägt ein Rändelrad 38 und das gegenüberliegende Ende einen Zahnring 39. Dieser ist ähnlich wie bei der Mutter 3, durch eine fräsende Bearbeitung der Hülsenstirnseite hergestellt. Eine Axialbewegung der Hülse 37 in Richtung auf den Griff 35 ist durch einen Anschlagflansch 39 begrenzt.

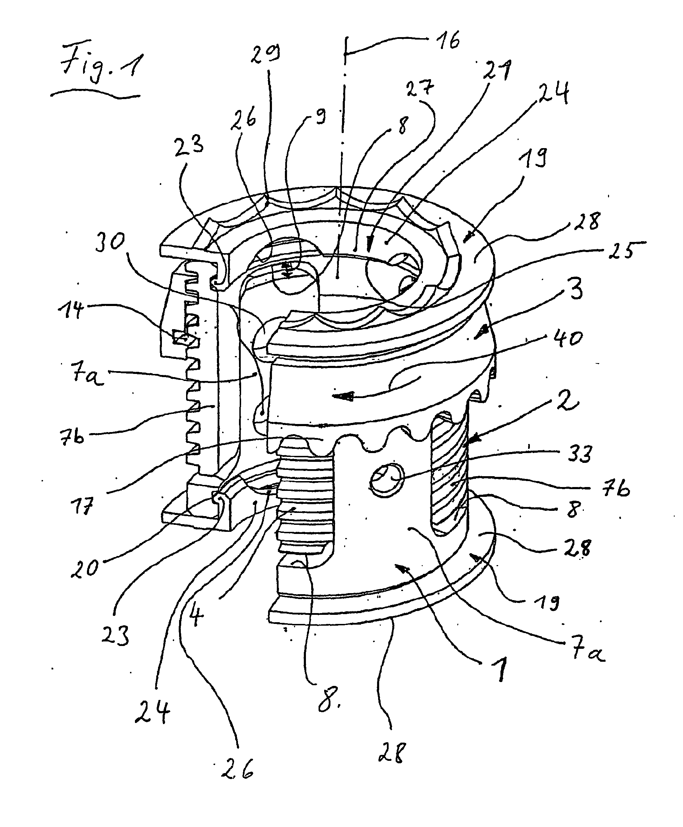

Zur Höhenverstellung des Implantats, beispielsweise ausgehend von der in Fig. 1 dargestellten Situation, wird das Handhabungswerkzeug'mit seinem Gewindeabschnitt 36 in die Gewindebohrung 33 eingedreht. Der Zahnring 39 steht dabei mit dem Zahnring 17 nach Art eines Kronenradgetriebes im Eingriff, d.h. die Achse des Zahnrings 39 verläuft im wesentlichen rechtwinklig zur Achse des Zahnrings 17. Das Rändelrad 38 liegt dabei am Anschlagflansch 29 an.

Nachdem das Implantat mit Hilfe des Handhabungswerkzeugs über eine Operationsöffnung in die Wirbelsäule eingesetzt wurde, wird zum Einstellen einer größeren Höhe des Implantats die Drehhülse 37 durch Betätigen des Rändelrades' 38 in Drehung versetzt. Wenn es sich bei dem Außengewinde 4 des Hülsenteils 2 und beim Innengewinde 10 der Mutter 3 um'Rechtsgewinde handelt, muss die Mutter 3 in Richtung des Pfeiles 40 und das Rändelrad in Richtung des Pfeiles 43 gedreht werden. Zum Lösen des Handhabungswerkzeuges vom Implantat wird dessen Gewindeabschnitt 36 durch Drehen des Griffs 35 aus der Gewindebohrung 33 entfernt. Da sich die Hülse 37 um den Stab 34 drehen kann, ist es nicht erforderlich, die Drehhülse während des Herausdrehens des Gewindeabschnittes 36 festzuhalten. Die Fixierung der einmal eingestellten Relativlage zwischen beiden Hülsenteilen 1,2 kann auf verschiedene Weise bewerkstelligt werden. Eine einfache Möglichkeit besteht darin, in die Gewindebohrung 33 eine etwa mit einer Spitze versehene Schraube (nicht dargestellt) einzudrehen.

### Bezugszeichenliste

- 1: erstes Hülsenteil
- 2: zweites Hülsenteil
- 3: Mutter
- 4: Außengewinde
- a,b: Stirnseite
- 6: Fenster
- 7a,b: Umfangsabschnitt
(a = erstes Hülsenteil;
b = zweites Hülsenteil)
- 8: Basis
- 9: Axialabstand
- 10: Innengewinde
- 13: Längsabschnitt
- 14: Ringnut
- 15: Vorsprung
- 16: Mittellängsachse
- 17: Zahnring
- 18: Stirnseite
- 19: Stirnplatte
- 20: Ringnut
- 21: Öffnung
- 23: Rastvorsprung
- 24: Schürze
- 25: Rand
- 26: Ausnehmung
- 27: Umfangsabschnitt
- 28: Außenseite
- 29: Zackenring
- 30: Durchbrechung
- 33: Gewindebohrung
- 34: Stab
- 35: Griff
- 36: Gewindeabschnitt
- 37: Drehhülse
- 38: Rändelrad
- 39: Anschlagflansch
- 40: Pfeil
- 43: Pfeil

## Patentansprüche

1. Höhenverstellbares Implantat zum Einsetzen zwischen Wirbelkörpern, mit einem ersten und einem zweiten Hülsenteil (1, 2), wobei das zweite Hülsenteil (2) ein Außengewinde (4) trägt, das mit einem Längsabschnitt im ersten Hülsenteil (1) drehfixiert und axial beweglich einliegt und an seinem aus dem ersten Hülsenteil (1) heraus ragenden Längsabschnitt von einer in das Außengewinde (4) eingreifenden Mutter (3) umfasst ist und die Mutter einen Zahnring (17) trägt, **dadurch gekennzeichnet, daß**
die Mutter (3) mit einem axial wirksamen Formschluss am ersten Hülsenteil (1) fixiert ist,
die Hülsenteile (1, 2) axial verlaufende, in ihren einander zugewandten Enden ausmündende Fenster (6) aufweisen, wobei die zwischen zwei benachbarten Fenstern angeordneten Umfangsabschnitte (7a, b) in den Fenstern (6) des jeweils anderen Hülsenteils (1, 2) axial verschiebbar einliegen,
die die beiden Hülsenteile (1, 2) umfassende Innenseite der Mutter einen oberen, mit einem Innengewinde (10) versehenen Längsabschitt und einen sich daran anschließenden gewindefrein Längsabschnitt (13) aufweist, in den eine Ringnut (14) eingearbeitet ist,
das erste Hülsenteil nahe seiner Stirnseite (5a) einen sich übe den Umfang des ersten Hülsenteils erstreckenden kreissegmentförmigen Vorsprung (15) aufweist, der in die Ringnut (14) der Mutter eingreift,
und wobei die Hülsenteile (1, 2) etwa die gleiche Wandstärke und den gleichen Innendurchmesser aufweisen.

2. Implantat nach Anspruch 1, **dadurch gekennzeichnet, dass** der Zahnring (17) an der dem ersten Hülsenteil (1) zugewandten Seite der Mutter (3) angeordnet ist.

3. Implantat nach Anspruch 2, **dadurch gekennzeichnet, dass** im ersten Hülsenteil (1) wenigstens eine radial verlaufende Gewindebohrung (33) zur Fixierung eines die Mutter (3) antreibenden Werkzeugs vorhanden ist.

4. Implantat nach einem der Ansprüche 1 bis 3, **dadurch gekennzeichnet, dass** an der zur Anlage an einem Wirbelkörper vorgesehenen Stirnseite (18) eines Hülsenteils (1, 2) eine als separates Teil ausgebildete Stirnplatte (19) lösbar fixiert ist.

5. Implantat nach Anspruch 4, **dadurch gekennzeichnet, dass** die Stirnplatte (19) mit Hilfe einer Schnappverbindung am Hülsenteil (1, 2) fixiert ist.

6. Implantat nach Anspruch 5, **gekennzeichnet durch** eine nahe der Stirnseite (18) angeordnete Ringnut (20) in der Innenwandung des Hülsenteils (1, 2), in die an der Stirnplatte (19) angeformte Rastnasen eingreifen.

## Claims

1. Height-adjustable implant for insertion between vertebral bodies, with a first and a second sleeve part (1, 2), which second sleeve part (2) has an external thread (4) and lies in the first sleeve part (1) by means of a longitudinal portion so as to be prevented from rotating and is axially displaceable, and its longitudinal portion projecting out from the first sleeve part (1) is enclosed by a nut (3) engaging in the external thread (4), and the nut bears a toothed ring (17), **characterised in that**
the nut (3) is secured on the first sleeve part (1) by an axially acting positive connection,
the sleeve parts (1, 2) have axially extending windows (6) opening at their mutually facing ends, and the peripheral portions (7a, b) disposed between two adjacent windows lie in the windows (6) of the other respective sleeve part (1, 2) so as to be axially displaceable,
the internal face of the nut enclosing the two sleeve parts (1, 2) has a top longitudinal portion provided with an internal thread (10) and a thread-free longitudinal portion (13) adjoining it, in which an annular groove (14) is formed,
the first sleeve part has a projection with the shape of a segment of a circle (15) close to its end face (5a) extending around the circumference of the first sleeve part, which engages in the annular groove (14) of the nut,
and the sleeve parts (1, 2) have approximately the same wall thickness and the same internal diameter.

2. Implant as claimed in claim 1, **characterised in that** the toothed ring (17) is disposed on the side of the nut (3) facing the first sleeve part (1).

3. Implant as claimed in claim 2, **characterised in that** at least one radially extending threaded bore (33) is provided in the first sleeve part (1) for securing a tool driving the nut (3).

4. Implant as claimed in one of claims 1 to 3, **characterised in that** an end plate (19) constituting a separate part is releasably fixed to the end face (18) of a sleeve part (1, 2) intended to be placed against a vertebral body.

5. Implant as claimed in claim 4, **characterised in that** the end plate (19) is secured to the sleeve part (1, 2) with the aid of a snap-fit connection.

6. Implant as claimed in claim 5, **characterised by** an annular groove (20) in the internal wall of the sleeve part (1, 2) disposed close to the end face (18) in which catch lugs integrally formed on the end plate (19) locate.

## Revendications

1. Implant réglable en hauteur destiné à être inséré entre des vertèbres, comportant une première et une deuxième partie formant manchon (1, 2), la deuxième partie formant manchon (2) étant munie d'un filetage extérieur (4), dont un tronçon longitudinal est inséré dans la première partie formant manchon (1) de manière immobile en rotation et de manière mobile dans le sens axial et qui, au niveau de son tronçon longitudinal en saillie hors de la première partie formant manchon (1), est entouré par un écrou (3) entrant en prise avec le filetage extérieur (4), et l'écrou portant une bague dentée (17), **caractérisé en ce que**
l'écrou (3) est fixé sur la première partie formant manchon au moyen d'un assemblage à correspondance de forme actif dans le sens axial,
les parties formant manchon (1, 2) comportent des fenêtres (6), orientées dans le sens axial et s'ouvrant au niveau de leurs extrémités orientées l'une vers l'autre, les tronçons périphériques (7a, b), agencés entre deux fenêtres voisines, étant insérés de manière mobile dans le sens axial dans les fenêtres (6) de respectivement l'autre partie formant manchon (1, 2),
le côté intérieur de l'écrou entourant les deux parties formant manchon (1, 2) présente un tronçon longitudinal muni d'un filetage interne (10) ainsi qu'un tronçon longitudinal (13) contigu qui est dépourvu du filetage, dans lequel est usinée une rainure annulaire (14),
la première partie formant manchon présente à proximité de sa face frontale (5a) une saillie (15) sous forme de segment de cercle qui s'étend sur la périphérie de la première partie formant manchon et qui entre en prise avec la rainure annulaire (14) de l'écrou,
et dans lequel les parties formant manchon (1, 2) présentent environ la même épaisseur de paroi ainsi que le même diamètre interne.

2. Implant selon la revendication 1, **caractérisé en ce que** la bague dentée (17) est située sur le côté de l'écrou (3) orienté vers la première partie formant manchon (1).

3. Implant selon la revendication 2, **caractérisé en ce qu'**il est prévu dans la première partie formant manchon (1) au moins une forure filetée (33), orientée dans le sens radial et destinée à la fixation d'un outil actionnant l'écrou (3).

4. Implant selon l'une quelconque des revendications 1 à 3, **caractérisé en ce qu'**une plaque frontale (19), conçue sous forme de pièce séparée, est fixée de manière amovible contre la face frontale (18) d'un partie formant manchon (1, 2), prévue pour venir en appui sur une vertèbre.

5. Implant selon la revendication 4, **caractérisé en ce que** la plaque frontale (19) est fixée contre le partie formant manchon (1, 2) au moyen d'un assemblage cliquet.

6. Implant selon la revendication 5, **caractérisé par** une rainure annulaire (20), qui est située à proximité de la face frontale (18) dans la paroi intérieure du partie formant manchon (1, 2) et dans laquelle s'engagent des ergots de blocage formés contre la plaque frontale (19).
